# EUROPÄISCHE PATENTSCHRIFT

(11) **EP 3 761 920 B1**
(45) Veröffentlichungstag und Bekanntmachung des Hinweises auf die Patenterteilung: **20.04.2022**
(21) Anmeldenummer: 19710372.4
(22) Anmeldetag: 07.03.2019
(51) Int. Cl.: A61F 5/01

(54) **ORTHESE MIT ANSCHLAGSTIFTHALTER**
ORTHOSIS HAVING A STOP PIN HOLDER
ORTHÈSE À SUPPORT DE GOUPILLE DE BUTÉE

(30) Priorität: 09.03.2018 DE 102018105480
(43) Veröffentlichungstag der Anmeldung: 13.01.2021
(73) Patentinhaber: Albrecht GmbH, 83071 Stephanskirchen (DE)
(72) Erfinder: OPAHLE, Hans-Georg, 83024 Rosenheim (DE)
(74) Vertreter: Flach Bauer Stahl Patentanwälte Partnerschaft mbB
(86) Internationale Anmeldenummer: PCT/EP2019/055693
(87) Internationale Veröffentlichungsnummer: WO 2019/170803

(56) Entgegenhaltungen:
- WO-A1-95/25489
- WO-A1-2017/050824
- US-A- 5 292 303
- US-A1- 2006 155 230

## Beschreibung

Orthopädische Orthesen werden bekannterweise dafür verwendet, Gelenke wie beispielsweise Knie- oder Ellbogengelenke zu führen und zu stabilisieren und/oder Gliedmaßen mittels Federkraft in Extensions-oder Flexionsrichtung zu drängen.

Eine Orthese gemäß dem Oberbegriff des Anspruchs 1 ist aus der WO 2017/050824 A1 bekannt. Bei Orthesen dieser Art sind die Schwenkbereichsgrenzen, innerhalb der die Schienenarme zwischen ihrer Extensions- und Flexionsendstellung relativ zueinander verschwenkt werden können, häufig mittels verstellbarer Anschlagstifte variabel einstellbar, die als Anschläge für den verschwenkbaren Schienenarm dienen. Üblicherweise werden diese Anschlagstifte in entsprechend angeordnete Bohrungen eingeschraubt.

Nachteilig ist bei diesen bekannten Orthesen, dass das Heraus- und Einschrauben der Anschlagstifte etwas umständlich ist und hierzu ein Werkzeug benötigt wird, das nicht immer griffbereit ist.

Aus der WO 95/25489 ist eine Orthese bekannt, bei der wahlweise ein Anschlag in einem bogenförmigen Schlitz eines Gehäuses installiert werden kann, um den Schwenkbereich eines Schienenarms um 15° zu verringern.

Der Erfindung liegt daher die Aufgabe zugrunde, eine Orthese der eingangs genannten Art mit einer Schwenkbereicheinstellvorrichtung zu schaffen, die einfach und werkzeuglos betätigbar ist und ein unverlierbares Halten der Schwenkbereich-Anschlagstifte sicherstellt.

Diese Aufgabe wird erfindungsgemäß durch eine Orthese mit den Merkmalen des Anspruchs 1 gelöst. Vorteilhafte Ausführungsformen der Erfindung sind in den weiteren Ansprüchen beschrieben.

Erfindungsgemäß umfasst die Schwenkbereicheinstellvorrichtung mindestens einen flexiblen Anschlagstifthalter, der einen um eine Achse drehbaren Lagerabschnitt und einen federnden Haltearm aufweist, an dem der Anschlagstift befestigt ist.

Die erfindungsgemäße Orthese bietet den Vorteil, dass die Verstellung der Schwenkbereichsgrenze(n) werkzeuglos und auf sehr einfache und schnelle Weise erfolgen kann. Besonders vorteilhaft ist ferner, dass der Anschlagstift unverlierbar an der Orthese gehalten wird. Zweckmäßigerweise ist hierbei der Anschlagstifthalter derart ausgebildet und angeordnet, dass der Anschlagstift nur gegen die Federkraft des Anschlagstifthalters aus einem der zur Begrenzung des Schwenkbereichs vorgesehenen Löcher herausgezogen werden kann. Weiterhin kann der Anschlagstifthalter auf sehr platzsparende Weise ausgebildet werden. Der erfindungsgemäße Anschlagstifthalter eignet sich daher in besonders guter Weise für Orthesen mit kleiner Baugröße, beispielsweise für Kinder oder Kleintiere.

Gemäß einer vorteilhaften Ausführungsform besteht der Lagerabschnitt aus einem Lagerring, der um die Schwenkachse der Orthese herum drehbar angeordnet ist. Hierdurch lässt sich der Anschlagstifthalter und damit der daran befestigte Anschlagstift auf sehr einfache Weise in diejenige Winkelposition um die Schwenkachse herum drehen, die für die gewünschte Positionierung des Anschlagstifts optimal ist, ohne dass es zu Spannungen innerhalb des Anschlagstifthalters in Umfangsrichtung kommen würde.

Vorteilhafterweise ist der Haltearm mäander-, S-, U-, V-oder W-förmig ausgebildet. Hierdurch lässt sich auf kleinstem Raum eine relativ große Länge des Haltearms erreichen. Dies schafft einen großen Bewegungsspielraum für den Anschlagstift, was das Entfernen des Anschlagstifts aus den Schwenkbereichbegrenzungslöchern und das Einsetzen in diese erleichtert.

Vorteilhafterweise weist der Haltearm einen ersten Armabschnitt, der konzentrisch zum Lagerring in einer ersten Umfangsrichtung verläuft, und einen zweiten Armabschnitt auf, der konzentrisch zum Lagerring in einer zweiten Umfangsrichtung verläuft, die zur ersten Umfangsrichtung entgegengesetzt ist. Die beiden Armabschnitte verlaufen dabei zweckmäßigerweise mit nur geringem Abstand zueinander kreisbogenförmig konzentrisch zur Schwenkachse der Orthese. Dies ermöglicht auf besonders platzsparende Weise den gewünschten Bewegungsspielraum für den Anschlagstift beim Verändern der Schwenkbereichsgrenze.

Vorteilhafterweise ist der Haltearm als Blattfeder ausgebildet. Zur Herstellung der Blattfeder kann ein sehr dünnes Federstahlblech mit einer Dicke von beispielsweise nur 0,2 - 0,5 mm, insbesondere 0,3 mm, verwendet werden, wodurch die Blattfeder nur sehr wenig Einbauhöhe beansprucht. Gleichzeitig sind derartige Blattfedern in Richtung ihrer Hauptebene formstabil, während sie senkrecht zur Hauptebene ein einfaches Ausfedern, d.h. ein leichtes elastisches Verbiegen, ermöglichen. Blattfedern aus einem anderen Material, beispielsweise Kunststoff, sind ebenfalls möglich.

Gemäß einer vorteilhaften Ausführungsform weist der Haltearm eine lösbare Stiftbefestigungseinrichtung in Form einer Clipverbindung zur Befestigung des Anschlagstifts auf. Hierdurch kann der Anschlagstift bei der Montage auf einfache und schnelle Weise am Anschlagstifthalter befestigt oder bei Bedarf von diesem demontiert werden. Alternativ ist es jedoch auch möglich, den Anschlagstift auf andere Weise, insbesondere durch eine Schweiß- oder Klebeverbindung, am Haltearm zu befestigen.

Vorteilhafterweise umfasst die Befestigungseinrichtung ein Einsteckloch zum Einstecken des Anschlagstifts und mindestens eine Feder- oder Rastzunge, die in eine Umfangsnut des Anschlagstifts eingreift. Besonders bevorzugt ist ein Federzungen- oder Rastzungenkranz vorgesehen, der sich um das Einsteckloch herum nach innen erstreckt und mit dem Anschlagstift verrastend in Eingriff tritt, wenn er in den Bereich der Umfangsnut des Anschlagstifts gelangt. Hierdurch wird der Anschlagstift auf besonders einfache und sichere Weise am Haltearm gehalten.

Vorzugsweise sind der Lagerabschnitt und der Haltearm als einstückiges Federblechelement ausgebildet. Hierdurch lässt sich der Anschlaghalter auf besonders einfache und kostengünstige Weise, beispielsweise mittels eines Stanzvorgangs, herstellen. Alternativ ist es auch möglich, den Lagerabschnitt und den Haltearm getrennt herzustellen und nachträglich miteinander zu verbinden.

Die Erfindung wird nachfolgend anhand der Zeichnungen beispielhaft näher erläutert. In diesen zeigen:
- Fig. 1:: eine Darstellung des Gelenkbereichs der erfindungsgemäßen Orthese ohne Deckel, wobei das Achselement geschnitten dargestellt ist,
- Fig. 2:: eine Explosionszeichnung der erfindungsgemäßen Orthese,
- Fig. 3: eine Draufsicht auf einen Anschlagstifthalter,
- Fig. 4:: eine Seitenansicht eines Anschlagstifts,
- Fig. 5:: eine Draufsicht auf den Anschlagstift von Fig. 4,
- Fig. 6:: eine frontseitige Ansicht des Achselements, und
- Fig. 7:: einen Längsschnitt durch das Achselement von Figur 6.

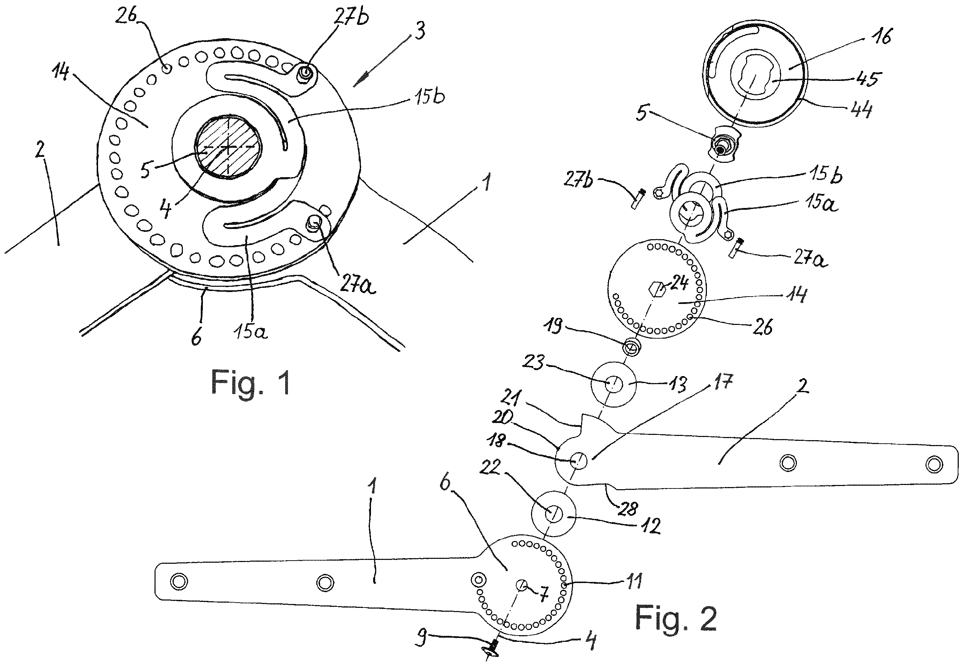

Figur 1 zeigt einen mittleren Abschnitt einer erfindungsgemäßen Orthese ohne Deckel mit einem ersten Schienenarm 1 und einem zweiten Schienenarm 2, wobei ein Achselement 5 geschnitten dargestellt ist. Die beiden Schienenarme 1, 2 sind lediglich verkürzt dargestellt und weisen nicht dargestellte Befestigungseinrichtungen, insbesondere Klettbänder, Schalen oder dergleichen, auf, mit denen die Schienenarme 1, 2 in bekannter Weise an mittels eines Körpergelenks verbundenen Gliedmaßen, beispielsweise am Ober- und Unterschenkel, Ober- und Unterarm etc., befestigt werden können.

Die Schienenarme 1, 2 sind in einem Gelenkbereich 3 gelenkig miteinander verbunden und um eine Schwenkachse 4 relativ zueinander schwenkbar. Die Schwenkachse 4 wird durch das Achselement 5 gebildet, das am ersten Schienenarm 1 festgelegt ist.

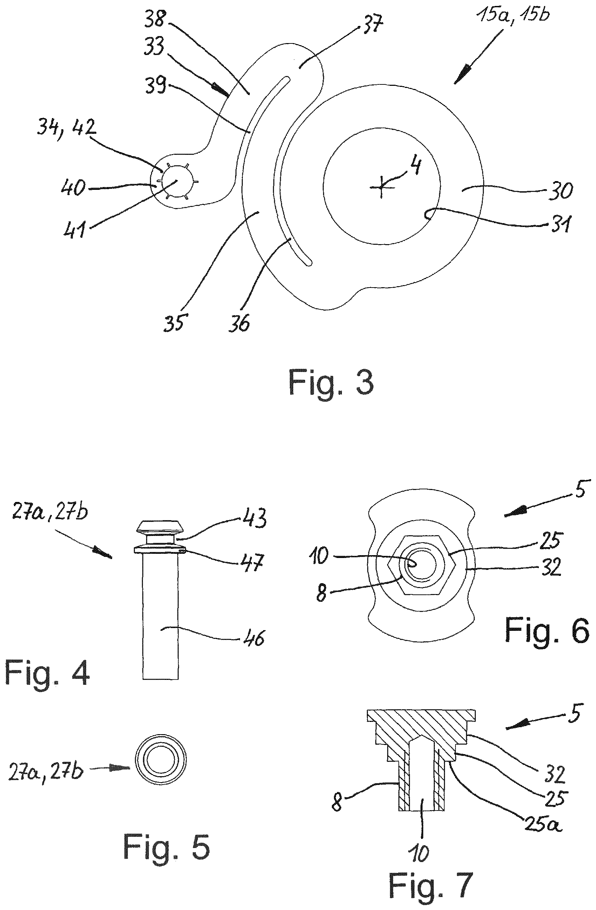

Figur 2 zeigt in Explosionsdarstellung den Aufbau der erfindungsgemäßen Orthese. Der erste Schienenarm 1 weist einen kreisscheibenförmigen Endbereich 6 mit einer zentralen Bohrung 7 auf. Diese Bohrung 7 dient zur Aufnahme eines zylinderförmigen Abschnitts 8 (Figur 7) des Achselements 5 und zum Hindurchführen einer zentralen Schraube 9, die in eine axiale Gewindebohrung 10 des Achselements 5 eingeschraubt werden kann, um das Achselement 5 und den ersten Schienenarm 1 drehfest miteinander zu verbinden.

In einem randnahen Bereich des kreisscheibenförmigen Endbereichs 6 ist eine Mehrzahl von Schwenkbereichsbegrenzungslöchern 11 vorgesehen, die auf einem zur Schwenkachse 4 konzentrischen Kreisbogen angeordnet sind.

In Axialrichtung aufeinanderfolgend weist die Orthese ferner eine erste Reibungsverminderungsscheibe 12, den zweiten Schienenarm 2, eine zweite Reibungsverminderungsscheibe 13, eine Lochscheibe 14, zwei Anschlagstifthalter 15a, 15b, das Achselement 5 und einen Deckel 16 auf.

Die beiden Reibungsverminderungsscheiben 12, 13 bestehen zweckmäßigerweise aus Kunststoff, insbesondere Teflon.

Der zweite Schienenarm 2 weist einen Endbereich 17 mit einer Bohrung 18 auf. Die Bohrung 18 dient zur Aufnahme einer zylindrischen Lagerhülse 19, die auf dem zylindrischen Abschnitt 8 des Achselements 5 drehbar gelagert ist, so dass der zweite Schienenarm 2 um die Schwenkachse 4 herum relativ zum ersten Schienenarm 1 verschwenkbar ist.

Der Endbereich 17 des zweiten Schienenarms 2 weist über etwa 180° einen kreisbogenförmigen Umfang 20 auf. An diesen kreisbogenförmigen Umfang 20 schließt sich eine radial vorstehende Anschlagfläche 21 an.

Die beiden Reibungsverminderungsscheiben 12, 13 weisen zentrale Löcher 22, 23 auf, die in gleicher Weise wie die Bohrung 18 zum Hindurchführen der Lagerhülse 19 dienen.

Die Lochscheibe 14 ist als Kreisscheibe ausgebildet und parallel zum Endbereich 6 des ersten Schienenarms 1 in einem derartigen feststehenden Abstand angeordnet, dass der zweite Schienenarm 2 dazwischen verschwenkt werden kann. Dieser Abstand wird durch die Länge der zylindrischen Lagerhülse 19 bestimmt, die mit einer Stirnseite an einer von einem Mehrkantabschnitt 25 des Achselements 5 gebildeten Durchmesserstufe 25a und mit ihrer anderen Stirnseite am ersten Schienenarm 1 anliegt. Die Lochscheibe 14 weist eine mittige Öffnung 24 mit einer mehreckigen Kontur auf, die zum drehfesten Aufstecken auf den entsprechend ausgebildeten Mehrkantabschnitt 25 des Achselements 5 dient. Weiterhin ist die Lochscheibe 14 mittels einer nicht dargestellten, in ihrem Randbereich angeordneten Schraubverbindung drehfest mit dem ersten Schienenarm 1 verbunden.

In einem randnahen Bereich der Lochscheibe 14 sind eine Mehrzahl von Schwenkbereichbegrenzungslöchern 26 vorgesehen, die zu den Schwenkbereichbegrenzungslöchern 11 des ersten Schienenarms 1 fluchten und wie diese auf einem zur Schwenkachse 4 konzentrischen Kreisbogen angeordnet sind. Die Schwenkbereichbegrenzungslöcher 11, 26 erstrecken sich im gezeigten Ausführungsbeispiel längs eines Kreisbogens über etwa 270° und können beispielsweise derart angeordnet sein, dass der Winkelabstand benachbarter Schwenkbereichbegrenzungslöcher 11, 26 jeweils 10° beträgt.

Die Schwenkbereichbegrenzungslöcher 11, 26 dienen zum Einstecken von zwei Anschlagstiften 27a, 27b zur Begrenzung des Schwenkbereichs der Orthese in Extensions- und Flexionsrichtung. Die Anschlagstifte 27a, 27b weisen hierzu eine zylindrischen Einsteckabschnitt 46 (Figur 4) auf, der parallel zur Schwenkachse 4 in ein fluchtendes Paar von Schwenkbereichbegrenzungslöchern 11, 26 bis zu einem radial vorspringenden Kragen 47 eingesteckt werden kann. Die Schwenkbereichsbegrenzung in Extensionsrichtung wird bewirkt, indem die Anschlagfläche 21 des zweiten Schienenarms 2 an einem der Anschlagstifte 27a, 27b anschlägt. Die Schwenkbereichsbegrenzung in Flexionsrichtung wird bewirkt, indem eine Anschlagfläche 28 des zweiten Schienenarms 2 am anderen Anschlagstift 27b, 27a anschlägt. Durch Einstecken der Anschlagstifte 27a, 27b in unterschiedliche Schwenkbereichbegrenzungslöcher 11, 26 können somit die Schwenkbereichsgrenzen der Orthese sowohl in Extensions- als auch in Flexionsrichtung verändert werden.

Die Anschlagstifte 27a, 27b werden jeweils durch einen separaten Anschlagstifthalter 15a, 15b unverlierbar am Achselement 5 gehalten. Im dargestellten Ausführungsbeispiel sind die beiden Anschlagstifthalter 15a, 15b gleich ausgebildet, jedoch spiegelbildlich gedreht am Achselement 5 angeordnet. Zweckmäßigerweise sind die beiden Anschlagstifthalter 15a, 15b in Axialrichtung der Orthese benachbart angeordnet.

Die Ausgestaltung der Anschlagstifthalter 15a, 15b geht aus Figur 3 hervor. Jeder Anschlagstifthalter 15a, 15b besteht aus einer ebenen, dünnen Blattfeder aus Federstahlblech, dessen Dicke zweckmäßigerweise etwa 0,2 bis 0,5 mm, insbesondere 0,3 mm, beträgt. Andere Materialien und Dicken sind möglich.

Weiterhin umfasst jeder Anschlagstifthalter 15a, 15b einen ringförmigen Lagerabschnitt 30 mit einer mittigen Öffnung 31 zum Hindurchführen eines zylinderförmigen Achsabschnitts 32 des Achselements 5 (Figur 7), auf dem der Anschlagstifthalter 15a, 15b drehbar gelagert ist. Die beiden Anschlagstifthalter 15a, 15b sind somit unabhängig voneinander um die Schwenkachse 4 drehbar.

Mit dem ringförmigen Lagerabschnitt 30 ist ein federnder, elastisch biegsamer Haltearm 33 verbunden, an dessen freiem Ende eine Stiftbefestigungseinrichtung 34 zur Befestigung des Anschlagstifts 27a, 27b vorgesehen ist.

Der Haltearm 33 umfasst einen ersten Armabschnitt 35, der konzentrisch zur Schwenkachse 4 in einer ersten Umfangsrichtung über einen Winkelbereich von etwa 110° verläuft. Der erste Armabschnitt 35 kann andere Längen aufweisen und sich beispielsweise über einen Winkelbereich von 30-180° erstrecken. Weiterhin ist der erste Armabschnitt 35 vom Lagerabschnitt 30 durch einen Spalt 36 getrennt, dessen Breite geringer als die Breite des ersten Armabschnitts 35 ist.

Der erste Armabschnitt 35 geht mittels eines 180°-Bogenabschnitts 37 in einen zweiten Armabschnitt 38 über, der ebenfalls konzentrisch zur Schwenkachse 4, jedoch in einer zweiten Richtung verläuft, die zur ersten Richtung gegenläufig ist. Die Breite des Spalts 39 zwischen dem zweiten Armabschnitt 38 und dem ersten Armabschnitt 35 ist wiederum geringer als die Breite der Armabschnitte 35, 38. Weiterhin erstreckt sich der zweite Armabschnitt 38 im gezeigten Ausführungsbeispiel über einen Winkelbereich von etwa 45-55°. Auch hier ist die Länge des zweiten Armabschnitts 38 in großem Umfang variabel.

Durch den mäanderförmigen Verlauf des Haltearms 33 kann dieser bei sehr kleiner Baugröße eine große Länge aufweisen. Hierdurch kann das freie Ende 4 des Haltearms 33, an dem der Anschlagstift 27a, 27b befestigt ist, ausreichend weit senkrecht zur Hauptebene des Anschlagstifthalters 15a, 15b hochgehoben werden, um den Anschlagstift 27a, 27b vollkommen aus den Schwenkbereichbegrenzungslöchern 11, 26 herausziehen und in einem gewünschten Schwenkbereichbegrenzungsloch 11, 26 einsetzen zu können. Da das Herausziehen der Anschlagstifte 27a, 27b aus den Schwenkbereichbegrenzungslöchern 11, 26 nur gegen die Federkraft der Anschlagstifthalter 15a, 15b möglich ist, verhindern die Anschlagstifthalter 15a, 15b gleichzeitig eine unerwünschte Axialverschiebung oder ein Lösen der Anschlagstifte 27a, 27b aus den Schwenkbereichbegrenzungslöchern 11, 26.

Die am freien Ende 40 des Haltearms 33 angeordnete Stiftbefestigungseinrichtung 34 ist im dargestellten Ausführungsbeispiel als lösbare Befestigungseinrichtung in Form einer Clipverbindung zur Befestigung des Anschlagstifts 27a, 27b ausgebildet. Hierzu weist die Stiftbefestigungseinrichtung 34 ein Einsteckloch 41 zum Einstecken des Anschlagstifts 27a, 27b und Feder- oder Rastzungen 42 auf, die in eine Umfangsnut 43 des Anschlagstifts 27a, 27b (Figur 4) eingreifen und dadurch den Anschlagstift 27a, 27b sowohl radial als auch axial halten. Wie in Figur 3 dargestellt, erstrecken sich die Feder- oder Rastzungen 42 um den gesamten Umfang des Einstecklochs 41 herum nach innen und können durch randseitige radiale Einschnitte in das Federstahlblech gebildet werden.

Der teller- bzw. napfförmige Deckel 16 kann derart ausgebildet sein, dass sich seine Umfangswand 44 über die Lochscheibe 14 bis nahe zum ersten Schienenarm 1 erstreckt. Eine seitliche Aussparung in der Umfangswand 44 schafft dabei den notwendigen Freiraum zum Verschwenken des zweiten Schienenarms 2 über einen maximalen Schwenkbereich von beispielsweise 120-160°, insbesondere von etwa 140° relativ zum ersten Schienenarm 1. Dieser maximale Schwenkbereich kann jedoch in weitem Umfang variieren.

Ferner kann der Deckel 16 auf unterschiedlichste Weise am Achselement 5, an der Lochscheibe 14 und/oder am ersten Schwenkarm 1 befestigt sein. Zweckmäßigerweise weist der Deckel 16 auf seiner Innenseite eine ebene Fläche 45 auf, die einen vorbestimmten, relativ geringen Abstand zur Lochscheibe 14 hat und die Lagerabschnitte 30 der beiden Anschlagstifthalter 15a, 15b mit geringem Spiel benachbart zur Lochscheibe 14 hält und die Anschlagstifthalter 15a, 15b in einer axialen Richtung festlegt.

Es ist ohne weiteres möglich, die beschriebene Schwenkbereicheinstellvorrichtung bei Orthesen zu verwenden, bei denen nur ein einzelner Anschlagstift 27a, 27b vorgesehen ist.

## Patentansprüche

1. Orthese mit einem ersten und zweiten Schienenarm (1, 2), die gelenkig miteinander verbunden und relativ zueinander innerhalb eines bestimmten Schwenkbereichs schwenkbar sind, und mit einer Schwenkbereicheinstellvorrichtung, die mindestens einen Anschlagstift (27a, 27b) zur Begrenzung des Schwenkbereichs aufweist, wobei der Anschlagstift (27a, 27b) wahlweise in unterschiedlich positionierten Schwenkbereichbegrenzungslöchern (11, 26) der Orthese anordenbar ist, **dadurch gekennzeichnet, dass** die Schwenkbereicheinstellvorrichtung mindestens einen flexiblen Anschlagstifthalter (15a, 15b) umfasst, der einen um eine Schwenkachse (4) drehbaren Lagerabschnitt (30) und einen federnden Haltearm (33) aufweist, an dem der Anschlagstift (27a, 27b) befestigt ist.

2. Orthese nach Anspruch 1, **dadurch gekennzeichnet, dass** der Lagerabschnitt (30) aus einem Lagerring besteht, der um die Schwenkachse (4) der Orthese herum drehbar angeordnet ist.

3. Orthese nach Anspruch 1 oder 2, **dadurch gekennzeichnet, dass** der Haltearm (33) mäander-, S-, U-, V- oder W-förmig ausgebildet ist.

4. Orthese nach Anspruch 2 oder 3, **dadurch gekennzeichnet, dass** der Haltearm (33) einen ersten Armabschnitt (35), der konzentrisch zum Lagerring in einer ersten Umfangsrichtung verläuft, und einen zweiten Armabschnitt (38) aufweist, der konzentrisch zum Lagerring in einer zweiten Umfangsrichtung verläuft, die zur ersten Umfangsrichtung entgegengesetzt ist.

5. Orthese nach einem der vorhergehenden Ansprüche, **dadurch gekennzeichnet, dass** der Haltearm (33) als Blattfeder ausgebildet ist.

6. Orthese nach einem der vorhergehenden Ansprüche, **dadurch gekennzeichnet, dass** der Haltearm (33) eine lösbare Stiftbefestigungseinrichtung (34) in Form einer Clipverbindung zur Befestigung des Anschlagstifts (27a, 27b) aufweist.

7. Orthese nach Anspruch 4, **dadurch gekennzeichnet, dass** die Stiftbefestigungseinrichtung (34) ein Einsteckloch (41) zum Einstecken des Anschlagstifts (27a, 27b) und mindestens eine Feder- oder Rastzunge (42) umfasst, die in eine Umfangsnut (43) des Anschlagstifts (27a, 27b) eingreift.

8. Orthese nach einem der vorhergehenden Ansprüche, **dadurch gekennzeichnet, dass** der Lagerabschnitt (30) und der Haltearm (33) als einstückiges Federblechelement ausgebildet sind.

## Claims

1. Orthosis comprising a first and second splint arm (1, 2) which are hingedly connected to one another and can be pivoted relative to one another within a specific pivot region, and comprising a pivot region adjustment apparatus which has at least one stop pin (27a, 27b) for limiting the pivot region, it being possible for the stop pin (27a, 27b) to be selectively arranged in differently positioned pivot region limiting holes (11, 26) of the orthosis, **characterised in that** the pivot region adjustment apparatus comprises at least one flexible stop pin holder (15a, 15b) which has a bearing portion (30) that can be rotated about a pivot axis (4) and a resilient holding arm (33) to which the stop pin (27a, 27b) is attached.

2. Orthosis according to claim 1, **characterised in that** the bearing portion (30) consists of a bearing ring which is mounted so as to be rotatable about the pivot axis (4) of the orthosis.

3. Orthosis according to either claim 1 or claim 2, **characterised in that** the holding arm (33) is designed in a meandering S, U, V or W shape.

4. Orthosis according to either claim 2 or claim 3, **characterised in that** the holding arm (33) has a first arm portion (35) which extends concentrically to the bearing ring in a first circumferential direction, and a second arm portion (38) which extends concentrically to the bearing ring in a second circumferential direction, which is opposite to the first circumferential direction.

5. Orthosis according to any of the preceding claims, **characterised in that** the holding arm (33) is designed as a leaf spring.

6. Orthosis according to any of the preceding claims, **characterised in that** the holding arm (33) has a detachable pin attachment device (34) in the form of a clip connection for attaching the stop pin (27a, 27b).

7. Orthosis according to claim 4, **characterised in that** the pin attachment device (34) comprises an insertion hole (41) for inserting the stop pin (27a, 27b) and at least one spring or detent tongue (42) which engages in a circumferential groove (43) of the stop pin (27a, 27b).

8. Orthosis according to any of the preceding claims, **characterised in that** the bearing portion (30) and the holding arm (33) are designed as a one-piece spring steel sheet element.

## Revendications

1. Orthèse pourvue d'une première et d'une deuxième branche d'attelle (1, 2), qui sont reliées ensemble de manière articulée et sont pivotables l'une par rapport à l'autre dans une zone de pivotement déterminée, et pourvue d'un dispositif d'ajustement de la zone de pivotement, qui présente au moins une goupille de butée (27a, 27b) permettant la délimitation de la zone de pivotement, où la goupille de butée (27a, 27b) peut être placée au choix dans des trous de délimitation de la zone de pivotement (11, 26) de l'orthèse positionnés de manière diverse, **caractérisée en ce que** le dispositif d'ajustement de la zone de pivotement comprend au moins un support de goupille de butée (15a, 15b) souple qui présente une partie de palier (30) rotative autour d'un axe de pivotement (4) et une attelle de maintien (33) élastique sur laquelle la goupille de butée (27a, 27b) est fixée.

2. Orthèse selon la revendication 1, **caractérisée en ce que** la partie de palier (30) est constituée d'une bague de palier qui est disposée autour de l'axe de pivotement (4) de l'orthèse en étant rotative autour.

3. Orthèse selon la revendication 1 ou la revendication 2, **caractérisée en ce que** l'attelle de maintien (33) est conçue en forme de méandres, de S, d'U, de V ou de W.

4. Orthèse selon la revendication 2 ou la revendication 3, **caractérisée en ce que** l'attelle de maintien (33) présente une première partie d'attelle (35), qui s'étend de manière concentrique par rapport à la bague de palier dans une première direction circonférentielle, et une deuxième partie d'attelle (38) qui s'étend de manière concentrique par rapport à la bague de palier dans une deuxième direction circonférentielle, qui est opposée à la première direction circonférentielle.

5. Orthèse selon l'une des revendications précédentes, **caractérisée en ce que** l'attelle de maintien (33) est conçue sous forme d'un ressort à lames.

6. Orthèse selon l'une des revendications précédentes, **caractérisée en ce que** l'attelle de maintien (33) présente un dispositif de fixation de goupille (34) amovible sous la forme d'une connexion par clip, pour la fixation de la goupille de butée (27a, 27b).

7. Orthèse selon la revendication 4, **caractérisée en ce que** le dispositif de fixation de goupille (34) comprend un orifice d'insertion (41) permettant l'insertion de la goupille de butée (27a, 27b) et au moins une languette de ressort ou de butée (42), qui se met en prise dans une rainure circonférentielle (43) de la goupille de butée (27a, 27b).

8. Orthèse selon l'une des revendications précédentes, **caractérisée en ce que** la partie de palier (30) et l'attelle de maintien (33) sont conçues en une pièce sous forme d'un élément en tôle élastique.
